Europäisches Patentamt

European Patent Office

Office européen des brevets

(11) Publication number: **0 119 076**
**B1**

## EUROPEAN PATENT SPECIFICATION

(45) Date of publication of patent specification: **31.05.89**

(21) Application number: **84301602.3**

(22) Date of filing: **09.03.84**

(51) Int. Cl.⁴: **C 12 N 5/02, C 12 M 3/02, G 01 N 33/86**

(54) A substrate comprising regenerated collagen fibrils.

(30) Priority: **10.03.83 JP 39597/83**

(43) Date of publication of application:
**19.09.84 Bulletin 84/38**

(45) Publication of the grant of the patent:
**31.05.89 Bulletin 89/22**

(84) Designated Contracting States:
**DE FR GB IT NL SE**

(56) References cited:
EP-A-0 023 607
EP-A-0 030 885
EP-A-0 097 907
WO-A-82/00660
FR-A-1 567 174
GB-A-2 059 991

(73) Proprietor: **KOKEN CO. LTD.**
**5-18 Shimoochiai 3-chome**
**Shinjuku-ku Tokyo (JP)**

(72) Inventor: **Miyata, Teruo**
**6-29 Shimoochiai 3-chome**
**Shinjuku-ku Tokyo (JP)**
Inventor: **Namiki, Shin'ichi**
**650 Ome**
**Ome-shi Tokyo (JP)**

(74) Representative: **Crisp, David Norman et al**
**D. YOUNG & CO. 10 Staple Inn**
**London, WC1V 7RD (GB)**

Courier Press, Leamington Spa, England.

## Description

The present invention relates to substrates comprising regenerated collagen fibrils and their use as substrates for cell culture and as carriers for measuring the adhesion activity of blood platelets.

The technology for utilizing useful substances obtained by culturing animal cells in large amounts and isolating the products has been watched with keen interest as one field of biotechnology. Also, in academic studies of artificial organs, research has been directed to artificial organs of the type that animal cells are incorporated therewith.

In both cases, it is important to carry out mass proliferation by culturing the cells removed from living bodies while maintaining the physiological properties of the cells. Collagen plays a vital function as a supporting substance, namely a substrate, for the cells which form the organs and tissues in the living body. Accordingly, it is believed that collagen is the best substrate for cell culture among known substances. In general, animal cells grow and proliferate while adhering to the substrate, and thus the existence of an effective substrate is essential for maintaining the activities of the cells. For mass culture and mass proliferation of animal cells, the use of a substrate as fine particles rather than as flat plates is especially advantageous since it is necessary for the substrate to have a very large surface area. Thus, recently, beaded fine particles or microsphere carriers have been employed for cell culture; for example, a beaded substrate comprising crosslinked dextran has been commercially developed for the mass culture of cells (see Catalogue Cytodex 1, "Beaded Microcarrier for Cell Culture" from Pharmacia Japan Co., Ltd.).

As indicated above, collagen provides a substrate for various types of cells in the living body and has been utilized as a substrate for cell culture. However, conventional collagen has only been used as a substrate when coated onto the surface of various plastics or glass articles, or has only been used for cell culture on the surface or inside of a collagen gel obtained by neutralizing an acid solution of collagen under physiological conditions.

We have now discovered that the mass culture of cells is facilitated by substantially increasing the substrate surface area to which cells can adhere by the formation of beaded collagen substrates, as compared with known substrates. Thus, according to one aspect, the invention provides a substrate in the form of beads or microspheres (hereinafter referred to as collagen beads) comprising irregularly entangled regenerated collagen fibrils each having a diameter of 10—1000 μm, with an aqueous solution between the regenerated collagen fibrils, the content of regenerated collagen fibrils being 20—0.01 wt.%

In a further aspect, the invention provides a process for the preparation of a substrate in the form of beads, said substrate comprising regenerated collagen fibrils with an aqueous solution between the regenerated collagen fibrils, which process comprises (1) dispersing an acidic aqueous collagen solution in a water-immiscible organic solvent into numerous droplets to form an emulsion, and then solidifying the droplets by addition of a water-miscible organic solvent and an alkali to the emulsion; or (2) dispersing a neutral aqueous collagen solution in a water-immiscible organic solvent into numerous droplets to form an emulsion at a temperature of 0°C—25°C, and then solidifying the droplets by raising the temperature of the emulsion to 30°C—40°C.

Embodiments of the invention are illustrated, by way of example, in the accompanying drawing in which:

Figure 1 is a scanning electron microscope photograph (Magnification: 8000) showing regenerated collagen fibrils contained in collagen beads obtained by the method of Example 1 below before the culture test; and

Figure 2 is a phase microscope photograph (magnification; 70) showing the particle structure of a collagen bead after use for the culture test in Example 1 below.

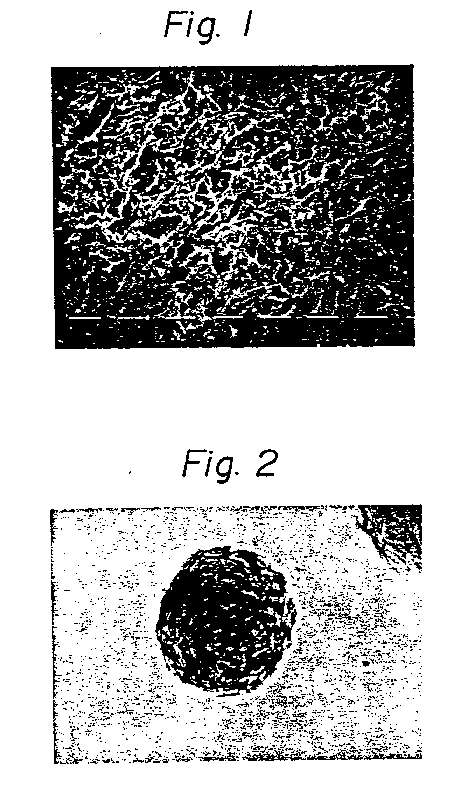

Collagen beads according to the invention contain 20—0.01 wt.% of regenerated collagen fibrils each having a diameter of 10—1000 μm. The regenerated collagen fibrils are irregularly entangled as, for example, can be seen in the scanning microscope picture of Figure 1. Between the regenerated collagen fibrils, there is an aqueous solution which can be replaced by any other water-miscible liquid. For example, when there is a physiological salt solution between the regenerated collagen fibrils, this is replaced, when the collagen beads are soaked in an aqueous medium for cell culture, by this aqueous medium. Collagen used according to the invention may be an acid-soluble collagen or a neutral salt-soluble collagen obtained by extracting collagen tissues of young animals with an aqueous neutral salt solution or an aqueous dilute acid solution.

Moreover, insoluble collagens exhibiting no solubility in these extraction procedures may be solubilized by treatment with a proteolytic enzyme such as pepsin to form an enzyme-solubilized collagen (atelocollagen), or by an alkali-treatment. These solubilized collagens may also be used according to the invention. Atelocollagen is a name given rather recently to a collagen from which telopeptides have been removed by treatment with proteolytic enzymes. The above mentioned insoluble collagens are insoluble owing to intermolecular crosslinkages formed through telopeptides at the ends of the molecules. When e.g. pepsin, a proteolytic enzyme, acts on the insoluble collagen, telopeptides alone are digested, and the intermolecular crosslinkages are broken and thus the insoluble collagen is solubilized to yield atelocollagen. Atelocollagen may also be obtained from soluble collagen extracted with an aqueous solution of dilute acid or neutral salt and by treatment with pepsin.

Water-immiscible organic solvents used according to the invention may include, for example, toluene, carbon tetrachloride, chloroform, cyclohexane, ether, petroleum ether, benzole and the like, as well as mixtures thereof. It is desirable to adjust, by using mixtures of the organic solvents, the specific gravity of the solvent to be as close as possible to the specific gravity of the aqueous collagen solution to be dispersed, in order to prevent floating or deposition of the aqueous collagen solution. For the formation of an emulsion by dispersing the acidic aqueous collagen solution into droplets, conventional methods may be used, e.g. by stirring or shaking the aqueous collagen solution in the water-immiscible organic solvent. The size of the dispersed droplets can be adjusted by controlling the degree of stirring or shaking. It is desirable that the amount of collagen solution used is equal to or less than that of the organic solvent. It is also desirable that the concentration of the aqueous collagen solution is less than about 5%, since a solution having a concentration above about 5% is generally too viscous, and thus difficult to disperse. In order to improve the stability of the resulting emulsion, it may be convenient to add a small amount of a surface active agent. Preferred surfactants which may be used are of the non-ionic type, for example, those of the SPAN-series (sorbitan fatty acid esters available from Atlas Powder Co. Ltd., U.S.A.) or the TWEEN-series (polyoxyethylene sorbitan fatty acid esters available from Atlas Powder Co. Ltd. U.S.A). The amount of surfactant used is preferably less than about 0.1% based on the total weight of the mixture of the organic solvent and the collagen solution.

Alkali-agents which may be used according to the invention for solidifying the droplets include basic substances such as ammonia, disodium hydrogen phosphate $(Na_2HPO_4)$, sodium hydroxide and the like. Water-miscible organic solvents which may be used for solidifying the droplets include methanol, ethanol, acetone and the like. The alkali agents and organic solvents may conveniently be added simultaneously to the emulsion, or the organic solvent may be added before the alkali is added. The amount of water-miscible organic solvent used is preferably above 50% based on the weight of the emulsion. The amount of alkali used may be that required to neutralize the droplets of dispersed collagen solution. A particularly preferred method of solidifying the droplets is to add a mixture of an aqueous base solution and a water-miscible organic solvent, to the emulsion. A preferred mixture is a mixture of aqueous ammonia and methanol, ethanol or acetone, the ammonia content conveniently being 1—2%. After the addition of the alkali and the water-miscible organic solvent, the emulsion can be slowly stirred for more than one hour, thereby solidifying the droplets of the aqueous solution of collagen. When working in this way, it is advantageous that a silicone oil has been previously applied to the walls of the reaction vessel used, thereby preventing the solidified collagen beads from adhering to the walls of the vessel.

For the formation of an emulsion by dispersing a neutral aqueous collagen solution into droplets, a similar dispersion procedure to that of the acidic collagen solution can generally be applied. A neutral aqueous collagen solution may be prepared by dissolving an acid soluble collagen or an atelocollagen in a buffer solution having a pH of 6.0—8.0, and an osmolarity of 200—400 mOsm/l at a temperature of 0°C—15°C. A phosphate buffer solution is preferably used for making the neutral aqueous collagen solution. The neutral aqueous collagen solution is conveniently added to a water-immiscible organic solvent containing a surfactant at a temperature of 0°C—25°C and stirred or shaken to form droplets. Even in a neutral aqueous collagen solution, a collagen concentration of less than 5% is desirable, since the viscosity of a solution having a concentration above 5% is very high. The amount of surfactant which can be used in the dispersion process is similar to that used in the acidic aqueous collagen solution.

In order to solidify the droplets of neutral aqueous collagen solution, the temperature of the mixture is raised to 30°C—40°C. The collagen solution of the droplets coagulates at a temperature of 30°C—40°C to form collagen fibril.

The solidified collagen beads prepared from the acidic or neutral aqueous collagen solution may preferably be crosslinked by hexamethylene diisocyanate (HMDIC) or glutaraldehyde. The crosslinking makes the beads stable to heat denaturation. In addition, while beads without crosslinking sometimes aggregate during storage, crosslinked beads are stable on storage. The solidified collagen beads prepared from the acidic or neutral aqueous collagen solution may be separated centrifugally or by using a screen, and they are preferably washed with a water-miscible organic solvent, such as methanol, ethanol, acetone or the like, followed by repeated water washing. The collagen beads thus obtained are somewhat non-uniform in particle size but it is possible to obtain fractions of uniform particle size such as by screening to obtain e.g. fractions over 48 mesh, between 48—100 mesh, between 100—200 mesh, and under 200 mesh.

The collagen beads according to the invention contain irregularly entangled regenerated collagen fibrils as mentioned above. By the use of the collagen beads suspended in a culture solution as substrates for cell culture, it is possible to have an enlarged area of substrate surface effective for cell adhesion, and the mass culture of cells may readily be achieved. When using an aqueous solution of collagen having a concentration, for example, of 1%, the finally obtained collagen beads after water washing have a collagen content of about 1%, and the remainder, 99%, comprises mainly water.

Accordingly, when the beads are equilibrated in a culture solution, a large proportion of the water in the beads can be replaced by the culture medium, and the specific gravity of the beads becomes approximately equal to that of the culture solution. The beads move slowly by slow stirring of the culture, with the result that the cells adhering to the beads are prevented from damage. The collagen beads according to the invention may also be used for measuring the adhesion activity of blood platelets. It is known that blood platelets adhere to collagen fibrils, and also cause a coagulating reaction. Accordingly, when a column is filled with collagen beads according to the invention, and a sample solution containing blood platelets (for example, blood admixed with anti-coagulants such as sodium citrate, EDTA; or platelet rich plasma) is passed through the column, it is possible to measure the platelet adhesion by counting the number of the blood platelets adhering to the collagen beads in the column. Based on this adhesion activity, it is possible to know the coagulative activity of the test blood, and it can be used in the diagnosis of diseases relating to blood, such as thrombosis. In the manufacture of collagen beads according to the invention, it is possible to obtain sterile beads under sterile conditions, while collagen beads manufactured under non-sterile conditions may be sterilized, for example, by placing them into a sealed vessel and irradiating with γ-rays of preferably 0.5—1.5 Mrad.

This invention will now be illustrated by the following non-limiting examples.

Example 1

Fresh dermis removed from a calf skin was finely divided with a micro-cutter manufactured by Stephan Co. Ltd. The finely divided powder was repeatedly washed with a 0.1 M aqueous solution of sodium acetate, followed by washing with water. The finely divided powder was then extracted with a 0.5 M aqueous solution of acetic acid, and the residual insoluble collagen was collected by filtration through a glass filter. The filtrate containing the acid soluble collagen was dialysed against a 0.02 M solution of disodium hydrogen phosphate ($Na_2HPO_4$) and thus the acid soluble collagen was precipitated as collagen fibrils. After repeatedly washing, the collagen fibrils were dissolved in 0.01 N HCl so that the collagen concentration was adjusted to 1 wt.%. The resulting acid-soluble collagen solution has a specific gravity of about 1.00. A mixed solvent was prepared by mixing toluene 800 ml and chloroform 220 ml in a vessel. The specific gravity of the mixed solvent was approximately equal to that of the above acid-soluble collagen solution. To the mixed solvent was added 0.1%, based on the weight of the mixed solvent, of Span 20 (a nonionic surface active agent of the sorbitan monolauric acid ester type available from Atlas Powder Co. Ltd.) and 300 ml of the above-mentioned 1% acid soluble collagen aqueous solution. Thereafter, the contents of the vessel were stirred vigorously for about 30 seconds, and then immediately one litre of ethanol containing 2% of ammonia was added. The contents of the vessel were stirred slowly for 2 hours, and the solidified collagen beads were obtained in a dispersed state. The liquid dispersion of the beads was filtered on a 200-mesh stainless steel screen. The beads obtained were soaked in 500 ml of ethanol and were filtered for washing the beads 3 times. These beads were further washed 3 times each with one litre of distilled water. The beads were then fractionated to determine the particle size distribution with stainless steel screens having openings of 48, 100 and 200 mesh. The results are given below, in terms of wt.%.

about 30%—oversize above 48 mesh
about 50%—fraction between 48—100 mesh
about 10%—fraction between 100—200 mesh
about 10%—undersize below 200 mesh

Figure 1 shows a scanning electron-microscope photograph of the structure of the collagen fibrils contained in the collagen beads. As can be seen from Figure 1, the regenerated collagen fibrils were irregularly entangled. The fractionated beads with different particle sizes were placed into sealed bottles and irradiated with γ-rays of 0.75 Mrad for sterilization. The collagen beads of the fraction between 48—100 mesh were used for a culture test of fibroblast of human dermis. The cells adhered to the beads after a few hours, and they proliferated so as to cover all the surface of the beads after 5 days. The beads were found to be an excellent substrate for the cells. Figure 2 shows a phase microscope photograph showing the particle structure of the collagen beads after use in the culture test.

Example 2:

100 g of the insoluble collagen obtained as the residue after extracting the acid soluble substance from the dermis of the calf skin in Example 1 were collected in a wet state, and one litre of 0.5 M acetic acid and 0.1 g of pepsin were added thereto. The resulting mixture was stirred at 20°C for 3 days. By this treatment, the insoluble collagen was dissolved to yield a viscous, pepsin-solubilized collagen (atelocollagen). After filtering the atelocollagen through a glass filter, the pH of the filtrate was adjusted to 7.5 by adding an aqueous sodium hydroxide solution, thereby forming fibrous precipitate. After separating in a centrifuge, the precipitate was washed with distilled water three times, and it was then dissolved in a sufficient amount of 0.01 N-HCl to give a solution containing 2% of collagen (specific gravity about 1.01). A mixed solvent (specific gravity: about 1.080) was prepared by mixing toluene (400 ml) and chloroform (115 ml), and to the solvent mixture was added a nonionic surface active agent, Tween 80 (nonionic surfactants of ethylene oxide condensate type of sorbitan mono-oleate available from Atlas Powder Co. Ltd.) in an amount of 0.1 wt% based on the weight of the solvent, and also 150 ml of the above-mentioned 2% collagen solution. The resulting solution was homogenized in a homogenizer with high-speed stirring at 10 000 r.p.m. for one minute, and it was immediately mixed with methanol containing 2 wt.% of ammonia and stirred slowly. As a result, solidified fine beads were obtained. These beads were fractionated using stainless wire screens, followed by washing with methanol three times and further with water three times. Similarly to Example 1, the particle size distribution was measured, and the results are given below, in terms of wt.%.

about 10%—oversize above 48 mesh
about 60%—fraction between 48—100 mesh
about 20%—fraction between 100—200 mesh
about 10%—undersize below 200 mesh

It was found that these beads are composed of collagen fibrils when observed in a scanning electron microscope. Each of the bead fractions was placed in a sealed glass bottle and irradiated with γ-rays of 1 Mrad for sterilization. The fraction of the beads between 48—100 mesh screen and fibroblasts of human dermis were used for suspension culture and these collagen beads proved to be an excellent substrate:

Example 3

Pepsin-solubilized collagen (atelocollagen) obtained by the same procedure as Example 2 was dissolved in 0.01 N-HCl to give a solution containing 1% of the collagen. The 1% atelocollagen solution was passed through an autoclave-sterilized filter having a fine pore diameter of 0.45 μm to give a sterile solution. To a mixed solvent of toluene (400 ml) with chloroform (110 ml) was added 0.1% of a nonionic surfactant, Span 20, and the mixed solvent was passed through a sterilized 0.22 μm microfilter for sterilization. To the resulting sterilized mixed solution was added 100 ml of sterilized 1% atelocollagen solution, and the solution wus stirred vigorously. Immediately thereafter, 500 ml of ethanol sterilized by being passed through a 0.22 μm diameter micropore filter was added to the solution. After stirring the solution slowly for 2 hours, 10 ml of aqueous 2%-ammonia were added to the solution, followed by stirring slowly for a further 2 hours. As a result, fine beads solidified in a dispersed state were obtained. The beads obtained were treated similarly to Example 1 and were washed with ethanol, followed by washing with water, and then fractionated. The results are given below, in terms of wt.%.

about 25%—oversize above 48 mesh
about 55%—fraction between 48—100 mesh
about 10%—fraction between 100—200 mesh
about 10%—undersize below 200 mesh

The collagen beads thus obtained were found to be made up of regenerated collagen fibrils when viewed in a scanning electron microscope, and were found to be an excellent substrate in a cell culture test.

Example 4

Atelocollagen obtained in the same way as in Example 2 was dissolved in 0.1 M phosphate buffer solution, pH 7.3, to give a collagen concentration of 1% at 10°C. The resulting neutral aqueous atelocollagen solution was added to a mixed solvent of toluene (400 ml) and chloroform (110 ml), containing 0.1% of Span 20 and stirred vigorously at 10°C. Immediately thereafter the mixture was warmed to 37°C and kept at 37°C for 2 hours to solidify the droplets. The solidified beads were washed with methanol to wash out toluene and chloroform, equilibrated with 0.9% NaCl solution and fractionated similarly to Example 1 with the results given below, in terms of wt.%.

about 25%—oversize above 48 mesh
about 60%—fraction between 48—100 mesh
about 12%—fraction between 100—200 mesh
about 3%—undersize below 200 mesh

The fractionated beads were irradiated with γ-rays at a total dose of 1.0 Mrad. The beads were found to be made up of regenerated collagen fibrils and to be an excellent substrate for cell culture.

Example 5

Atelocollagen beads were prepared using the same procedure as that of Example 4, but providing crosslinking with hexamethylene diisocyanate (HMDIC). After solidification of the droplets by raising the temperature to 37°C for 2 hours, the solidified beads were collected by screening through a 200 mesh screen, washed with methanol to remove toluene and chloroform, and thereafter soaked in 0.1% HMDIC in methanol solution for crosslinking at room temperature for 1 hour. After washing with methanol, the beads were equilibrated with 0.9% NaCl aqueous solution and fractionated on screens. The results are given below, in terms of wt.%.

about 20%—oversize above 48 mesh
about 50%—fraction between 48—100 mesh
about 20%—fraction between 100—200 mesh
about 10%—undersize below 200 mesh

The crosslinked, fractionated beads were γ-irradiated at 1.0 Mrad for sterilization. The beads thus prepared consisted of regenerated collagen fibrils, and were found to be an excellent substrate in a cell culture test.

**Claims**

1. A substrate in the form of beads comprising irregularly entangled regenerated collagen fibrils having a diameter of 10—1000 μm with an aqueous solution between the regenerated collagen fibrils, the content of the regenerated collagen fibrils being 20—0.01 wt.%.

2. A substrate according to claim 1 wherein the aqueous solution comprises an aqueous cell culture medium.

3. A process for the preparation of a substrate in the form of beads, said substrate comprising regenerated collagen fibrils with an aqueous solution between the regenerated collagen fibrils, which process comprises dispersing an acidic aqueous collagen solution in a water-immiscible organic solvent into numerous droplets to form an emulsion, and then solidifying the droplets by addition of a water-miscible organic solvent and an alkali to the emulsion.

4. A process according to claim 3 wherein the addition of the water-miscible organic solvent and the alkali is carried out simultaneously.

5. A process according to claim 3 wherein the addition of the water-miscible organic solvent is carried out before the addition of the alkali.

6. A process for the preparation of a substrate in the form of beads, said substrate comprising regenerated collagen fibrils with an aqueous solution between the regenerated collagen fibrils, which process comprises dispersing a neutral aqueous collagen solution in a water-immiscible organic solvent into numerous droplets to form an emulsion at a temperature of 0°C—25°C, and then solidifying the droplets by raising the temperature of the emulsion to 30°C—40°C.

7. A process according to any one of claims 3 to 5, wherein the solidified droplets are subjected to treatment with a crosslinking agent selected from hexamethylene diisocyanate and glutaraldehyde.

8. A process according to any one of claims 3 to 7 wherein the content of the regenerated collagen fibrils in the solidified droplets is 20—0.01 wt.%.

9. A method of culturing cells using a substrate as defined in claim 1.

10. A method of measuring the adhesion activity of blood platelets using a substrate as defined in claim 1.

**Patentansprüche**

1. Substrat in Form von Kügelchen enthaltend unregelmäßig verwickelte, regenerierte Kollagenfibrillen mit einem Durchmesser von 10—1000 µm mit einer wässrigen Lösung zwischen den regenerierten Kollagenfibrillen, wobei der Gehalt an den regenerierten Kollagenfibrillen 20—0,01 Gew.-% beträgt.

2. Substrat nach Anspruch 1, worin die wässrige Lösung ein wässriges Zellkulturmedium umfaßt.

3. Verfahren zur Herstellung eines Substrats in Form von Kügelchen, welches Substrat regenerierte Kollagenfibrillen mit einer wässrigen Lösung zwischen den regenerierten Kollagenfibrillen umfaßt, welches darin besteht, eine saure wässrige Kollagenlösung in einem mit Wasser nicht mischbaren organischen Lösungsmittel unter Bildung einer Emulsion zu zahlreichen Tröpfchen zu dispergieren und anschließend die Tröpfchen durch Zugeben eines mit Wasser mischbaren organischen Lösungsmittels und einer Base zu der Emulsion zu verfestigen.

4. Verfahren nach Anspruch 3, worin die Zugabe des mit Wasser mischbaren organischen Lösungsmittels und der Base gleichzeitig erfolgt.

5. Verfahren nach Anspruch 3, worin die Zugabe des mit Wasser mischbaren organischen Lösungsmittels vor der Zugabe der Base erfolgt.

6. Verfahren zur Herstellung eines Substrats in Form von Kügelchen, welches Substrat regenerierte Kollagenfibrillen mit einer wässrigen Lösung zwischen den regenerierten Kollagenfibrillen umfaßt, welches darin besteht, eine neutrale wässrige Kollagenlösung bei einer Temperatur von 0°C—25°C in einem mit Wasser nicht mischbaren organischen Lösungsmittel unter Bildung einer Emulsion zu zahlreichen Tröpfchen zu dispergieren und anschließend die Tröpfchen durch Erhöhen der Emulsionstemperatur auf 30°C—40°C zu verfestigen.

7. Verfahren nach einem der Ansprüche 3 bis 5, worin die verfestigten Tröpfchen einer Behandlung mit einem Vernetzungsmittel, das aus der Hexamethylendiisocyanat und Glutaraldehyd umfassenden Gruppe ausgewählt ist, unterworfen werden.

8. Verfahren nach einem der Ansprüche 3 bis 7, worin der Gehalt an den regenerierten Kollagenfibrillen in den verfestigten Tröpfchen 20—0,01 Gew.-% beträgt.

9. Verfahren zum Kultivieren von Zellen unter Verwendung eines Substrats nach Anspruch 1.

10. Verfahren zum Messen der Haftungsaktivität von Blutplättchen unter Verwendung eines Substrats nach Anspruch 1.

**Revendications**

1. Un substrat sous forme de pertes comprenant des fibrilles de collagène régénéré enchevêtrées de manière irrégulière ayant un diamètre de 10—1 000 µm avec une solution aqueuse incluse entre les fibrilles de collagère régénéré, la teneur en fibrilles de collagène régénéré étant de 20—0,01% en poids.

2. Un substrat selon la revendication 1, selon lequel la solution aqueuse comprend un milieu aqueux de culture cellulaire.

3. Un procédé de préparation d'un substrat sous la forme de perles, ledit substrat comprenant des fibrilles de collagène régénéré avec une solution aqueuse incluse entre les fibrilles de collagène régénéré, ledit procédé comprend la dispersion d'une solution aqueuse acide de collagène dans un solvant organique non miscible à l'eau en gouttelettes nombreuses pour former une émulsion et ensuite la solidification des gouttelettes par addition d'un solvant organique miscible à l'eau et d'un alcali à l'émulsion.

4. Un procédé selon la revendication 3, selon lequel l'addition du solvant organique miscible à l'eau et de l'alcali est conduite simultanément.

5. Un procédé selon la revendication 3, selon lequel l'addition du solvant organique miscible à l'eau est conduit avant l'addition de l'alcali.

6. Un procédé de préparation d'un substrat sous la forme de perles, ledit substrat comprenant des fibrilles de collagène régénéré avec une solution aqueuse incluse entre les fibrilles de collagène régénéré, ledit procédé comprenant la dispersion d'une solution aqueuse neutre de collagène dans un solvant organique non miscible à l'eau en gouttelettes nombreuses pour former une émulsion à une température de 0°C—25°C, et ensuite la solidification des gouttelettes par élévation de la température de l'émulsion à 30°C—40°C.

7. Un procédé selon l'une quelconque des revendications 3 à 5, selon lequel les gouttelettes solidifiées sont soumises à un traitement par un agent de réticulation choisi parmi le diisocyanate d'hexaméthylène et le glutaraldéhyde.

8. Un procédé selon l'une quelconque des revendications 3 à 7, selon lequel la teneur en fibrilles de collagène régénéré dans les gouttelettes solidifiées est de 20—0,01% en poids.

9. Une méthode de culture cellulaire utilisant un substrat tel que défini dans la revendication 1.

10. Une méthode de mesure de l'activité adhésive des plaquettes sanguines utilisant un substrat tel que défini dans la revendication 1.

*Fig. 1*

*Fig. 2*